# EUROPEAN PATENT SPECIFICATION

(11) **EP 2 791 682 B1**
(45) Date of publication and mention of the grant of the patent: **30.09.2015**
(21) Application number: 12806633.9
(22) Date of filing: 10.12.2012
(51) Int. Cl.: G01N 33/68

(54) **ASSAY**
TEST
DOSAGE

(30) Priority: 12.12.2011 GB 201121265
(43) Date of publication of application: 22.10.2014
(73) Proprietor: The Binding Site Group Limited, Birmingham, West Midlands B15 1QT (GB)
(72) Inventor: HARDING, Stephen, Birmingham West Midlands B15 1QT (GB); HUGHES, Richard, Birmingham West Midlands B15 1QT (GB); HUTCHISON, Colin, Birmingham West Midlands B15 1QT (GB)
(74) Representative: Elsy, David
(86) International application number: PCT/GB2012/053071
(87) International publication number: WO 2013/088126

(56) References cited:
- WO-A1-2011/114150
- AQUI NICOLE A ET AL: "Use of serum protein electrophoresis to monitor patients with post-transplant lymphoproliferative disorder", AMERICAN JOURNAL OF TRANSPLANTATION, BLACKWELL MUNKSGAARD, DK, vol. 3, no. 10, 1 October 2003 (2003-10-01), pages 1308-1311, XP009167203, ISSN: 1600-6135
- CAPELLO DANIELA ET AL: "Analysis of immunoglobulin heavy and light chain variable genes in post-transplant lymphoproliferative disorders", HEMATOLOGICAL ONCOLOGY, WILEY, CHICHESTER, US, vol. 24, no. 4, 1 December 2006 (2006-12-01), pages 212-219, XP009167215, ISSN: 0278-0232 [retrieved on 2006-08-09]
- AGBALIKA FELIX ET AL: "Epstein-Barr virus early-antigen antibodies before allogeneic haematopoietic stem cell transplantation as a marker of risk of post-transplant lymphoproliferative disorders", BRITISH JOURNAL OF HAEMATOLOGY, WILEY-BLACKWELL PUBLISHING LTD, GB, vol. 136, no. 2, 1 January 2007 (2007-01-01), pages 305-308, XP009167204, ISSN: 0007-1048 [retrieved on 2006-11-23]
- ENGELS E A ET AL: "Circulating antibody free light chains and risk of posttransplant lymphoproliferative disorder", AMERICAN JOURNAL OF TRANSPLANTATION, WILEY INTERSCIENCE, vol. 12, no. 5, 1 May 2012 (2012-05-01), pages 1268-1274, XP009167208, ISSN: 1600-6143, DOI: 10.1111/J.1600-6143.2011.03954.X

## Description

The invention relates to methods of identifying the risk of an organ transplant patient developing or having post transplant lymphoproliferative disorder (PTLD) and monitoring the development of or treatment of PTLD, and assay kits for the methods.

The occurrence of post transplant lymphoproliferative disorder (PTLD) following solid organ transplant is reported in the literature to vary from 1 - 30%. The incidence is dependent on the type of solid organ transplant and age of the individual involved. As high as 85% of PTLD is B-cell in origin and is predominantly associated with EBV (Epstein-Barr Virus) infection. Although PTLD may occur at any point post transplant it is thought that the risk is highest in the first year following the transplant.

| Organ Transplant | Adults (% incidence) | Paediatric (% incidence) |
|---|---|---|
| Kidney | 1-2.3 | 1.2-10.1 |
| Liver | 1-2.8 | 4-15 |
| Heart | 1-6.3 | 6.4-19.5 |
| Heart/Lung | 2.4-5.8 | 6.4-19.5 |
| Lung | 4.2-10 | 6.4-19.5 |
| Small Bowel | 20 | 30 |

| | | |
|---|---|---|
| (Parker et al. "Diagnosis of post-transplant lymphoproliferative disorder in solid organ transplant recipients - BCSH and BTS Guidelines." Br J Haematol 149.5 (2010) 675-92) | | |

Solid organ transplant patients are heavily immune-suppressed in order to reduce the risk of organ rejection. However, due to the reduced T-cell surveillance that this causes this also increases the risk of Epstein Barr virus (EBV) reactivation (a known cause of B-cell transformation). EBV is a herpes virus that is found in ∼95% of the population and not normally considered a problem. It is believed by the Applicant that the reactivation is followed by B-cell proliferation and in certain cases leading, eventually, to PTLD. When PTLD is identified the immediate response is to reduce immune-suppression (immune-suppression is frequently T-cell targeted in transplant patients), which in certain cases is thought to resolve the PTLD.

EBV is a transforming agent known to result in immortalisation and uncontrolled proliferation of B-cells. Normally this is controlled by a T-cell response, however transplant patients are usually immune-suppressed.

EBV associated PTLD refers to all EBV driven lymphoproliferation, including lymphomas but also post-transplant infectious mononucleosis, itself if treated less of a concern. Cytomegalo virus (CMV) has also been associated with PTLD. In around 20% of cases the PTLD is not associated with EBV infection, here the PTLD is often reported to occur much later after transplant.

There are four main types:
a) Early lesions - Plasmacytic hyperplasia
b) Polymorphic PTLD - Destructive polyclonal or monoclonal expansion
c) Monomorphic
   a. B-cell PTLD - Diffuse Large B-Cell Lymphoma (DLBCL) (most common), Burkitt lymphoma, plasma cell myeloma
   b. T-cell PTLD (rare)
d) Classical Hodgkin lymphoma type PTLD (very rare)

The Applicant has realised that PTLD appears to correlate with uncontrolled proliferation of B cells and related EBV infection. Free light chains (FLC) are produced by B cells resulting in elevation of many cases. FLCs are relatively easy to detect and measure thus allowing an assay for PTLD to be produced.

Landgren et al. (J. Clin. Oncol., 2010, 28, 1-7), showed that elevated free light chain levels in HIV is associated with an increased risk of HIV associated lymphoma up to 5 years prior to diagnosis of the lymphoma in HIV patients. In addition, both abnormal FLC ratios and elevated levels have been reported to occur in lymphoma patients. Furthermore, Maurer et al. (J. Clin. Oncol., 2011, 29, 1-11) showed that in non-PTLD associated DLBCL that elevated FLCs at diagnosis show inferior overall survival as well as poorer event free survival (HR 3.7 & 2.6).

Antibodies comprise heavy chains and light chains. They usually have a two-fold symmetry and are composed of two identical heavy chains and two identical light chains, each containing variable and constant region domains. The variable domains of each light-chain/heavy-chain pair combine to form an antigen-binding site, so that both chains contribute to the antigen-binding specificity of the antibody molecule. Light chains are of two types, κ and λ and any given antibody molecule is produced with either light chain but never both. There are approximately twice as many κ as λ molecules produced in humans, but this is different in some mammals. Usually the light chains are attached to heavy chains. However, some unattached "free light chains" are detectable in the serum or urine of individuals. Free light chains may be specifically identified by raising antibodies against the surface of the free light chain that is normally hidden by the binding of the light chain to the heavy chain. In free light chains (FLC) this surface is exposed, allowing it to be detected immunologically. Commercially available kits for the detection of κ or λ free light chains include, for example, "Freelite™", manufactured by The Binding Site Limited, Birmingham, United Kingdom. The Applicants have previously identified that determining the amount of free κ/free λ ratios, aids the diagnosis of monoclonal gammopathies in patients. It has been used, for example, as an aid in the diagnosis of intact immunoglobulin multiple myeloma (MM), light chain MM, non-secretory MM, AL amyloidosis, light chain deposition disease, smouldering MM, plasmacytoma and MGUS (monoclonal gammopathies of undetermined significance). Detection of FLC has also been used, for example, as an aid to the diagnosis of other B-cell dyscrasia and indeed as an alternative to urinary Bence Jones protein analysis for the diagnosis of monoclonal gammopathies in general.

FLCs (Bence Jones Proteins) have been previously monitored in transplant patients. However, this has been in the urine of patients to assess the health of transplanted kidneys (US 2003/0232396). Damaged kidneys often result in the release of increased amount of FLC into the urine.

The invention provides a method of identifying the risk of an organ transplant, patient developing post transplant lymphoproliferative disorder (PTLD), identifying the likelihood of having PTLD, or prognosis of a patient with PTLD, comprising detecting an amount of free light chains (FLC) in a sample from the patient, wherein a higher than normal amount of FLC or abnormal FLC ratio in the sample, indicates an increased risk of developing PTLD, having PTLD or having a poor prognosis for the PTLD.

A further aspect of the invention provides a method of monitoring the progress of PTLD in a patient, monitoring treatment of a patient with increased risk of developing PTLD, or monitoring the treatment of PTLD in a patient comprising detecting an amount of FLC in a sample from the patient and comparing it to an amount of FLC from a previously taken sample from the patient.

The method may be implemented by determining an amount of the amount of FLC in the sample from the patient, inputting that amount into a computer, wherein the computer comprises a value for an amount of FLC recorded from a predetermined normal amount of FLC or the previously taken sample from the patient, comparing the two values using the computer and generating a computer output showing the different in the amount of FLC detected and/or generating a computer output indicating a risk of PTLD.

The computer may comprise a computer input for inputting the amounts of FLC, a computer memory adapted to compare the two amounts of FLC, and a computer output for indicating the difference in the amounts of FLC and/or generating output of the risk of PTLD.

The product of the method (the result of the assay) may then be sent to a physician to make a final assessment of the clinical significance of the result.

The organ transplant may be a solid organ such as a heart, lung, liver, kidney, bowel, face, skin or other transplant.

The methods may be combined with, for example, tissue biopsies to allow the histology of the PTLD to be studied.

The PTLD may be an early lesion, polymorphic PTLD, monomorphic PTLD, or classical Hodgkin Lymphoma type as defined above.

The patient has typically had an organ transplant.

The amount of FLC detected may be one or more of:
(i) the amount of κ FLC
(ii) the amount of λ FLC
(iii) the total amount of FLC (κ+λ) and/or
(iv) the ratio of the amount of κ: λ in the sample.

Assessing the risk of PTLD in patients following organ transplant, for example by looking at elevated FLC levels (κ, λ or total FLC), indicates increased risk of PTLD and helps identify those patients who will benefit from closer management of their immunosuppressive treatment.

The method therefore typically includes the step of changing the immuno-suppressive treatment regime of the patient.

Following changes in treatment following identification of patients with increased risk, for example, identified by the method of the first aspect of the invention, or patients with PTLD, allows monitoring of treatment. A decrease in FLC indicates, for example, a positive response to treatment.

Assessing the prognosis of PTLD allows clinical decisions to be made on the choice of, for example, aggressive or non-aggressive treatments. This may be assessed, for example, by abnormal FLC ratios or an elevated level of FLCs (κ, λ or total FLC).

The amount of FLCs are normally compared to predefined normal FLC levels (for example an elevation above 50 mg/L, though this may be lower in immune-suppressed patients). Normal ranges for κ/λ ratios are 0.26 - 1.65 mg/L.

Preferably prior to the transplant the patients do not have symptoms of a B-cell associated disease. The symptoms may include recurrent infections, bone pain and fatigue. Such a B-cell associated disease is preferably not a myeloma, (such as intact immunoglobulin myeloma, light chain myeloma, non-secretory myeloma), an MGUS, AL amyloidosis, Waldenström's macroglobulinaemia, Hodgkin's lymphoma, follicular centre cell lymphoma, chronic lymphocytic leukaemia, mantle cell lymphoma, pre-B cell leukaemia or acute lymphoblastic leukaemia. Moreover, the individual typically does not have reduced bone marrow function.

The term "total free light chains" means the amount of κ and λ free light chains in the sample from the patient.

The sample is typically a sample of serum from the subject. However, whole blood, plasma, urine or other samples of tissue or fluids may also potentially be utilised.

Typically the FLC, such as total FLC, is determined by immunoassay, such as ELISA assays, Serum Protein Electrophoresis (SPE), or utilising fluorescently labelled beads, such as Luminex ™ beads.

The antibodies used to determine the amount of FLC may be whole or fragments of antibodies specific for FLC. They may be polyclonal or monoclonal.

Sandwich assays, for example use antibodies to detect specific antigens. One or more of the antibodies used in the assay may be labelled with an enzyme capable of converting a substrate into a detectable analyte. Such enzymes include horseradish peroxidase, alkaline phosphatase and other enzymes known in the art. Alternatively, other detectable tags or labels may be used instead of, or together with, the enzymes. These include radioisotopes, a wide range of coloured and fluorescent labels known in the art, including fluorescein, Alexa fluor, Oregon Green, BODIPY, rhodamine red, Cascade Blue, Marina Blue, Pacific Blue, Cascade Yellow, gold; and conjugates such as biotin (available from, for example, Invitrogen Ltd, United Kingdom). Dye sols, chemiluminescent labels, metallic sols or coloured latex may also be used. One or more of these labels may be used in the ELISA assays according to the various inventions described herein or alternatively in the other assays, labelled antibodies or kits described herein.

The construction of sandwich-type assays is itself well known in the art. For example, a "capture antibody" specific for the FLC is immobilised on a substrate. The "capture antibody" may be immobilised onto the substrate by methods which are well known in the art. FLC in the sample are bound by the "capture antibody" which binds the FLC to the substrate via the "capture antibody".

Unbound immunoglobulins may be washed away.

In ELISA or sandwich assays the presence of bound immunoglobulins may be determined by using a labeled "detecting antibody" specific to a different part of the FLC of interest than the binding antibody.

Flow cytometry may be used to detect the binding of the FLC of interest. This technique is well known in the art for, e.g. cell sorting. However, it can also be used to detect labeled particles, such as beads, and to measure their size. Numerous text books describe flow cytometry, such as Practical Flow Cytometry, 3rd Ed. (1994), H. Shapiro, Alan R. Liss, New York, and Flow Cytometry, First Principles (2nd Ed.) 2001, A.L. Given, Wiley Liss.

One of the binding antibodies, such as the antibody specific for FLC, is bound to a bead, such as a polystyrene or latex bead. The beads are mixed with the sample and the second detecting antibody. The detecting antibody is preferably labeled with a detectable label, which binds the FLC to be detected in the sample. This results in a labeled bead when the FLC to be assayed is present.

Other antibodies specific for other analytes described herein may also be used to allow the detection of those analytes.

Labeled beads may then be detected via flow cytometry. Different labels, such as different fluorescent labels may be used for, for example, the anti- free λ and anti- free κ antibodies. Other antibodies specific for other analytes, EBV described herein may also be used in this or other assays described herein to allow the detection of those analytes. This allows the amount of each type of FLC bound to be determined simultaneously or the presence of other analytes to be determined.

Alternatively, or additionally, different sized beads may be used for different antibodies, for example for different marker specific antibodies. Flow cytometry can distinguish between different sized beads and hence can rapidly determine the amount of each FLC or other analyte in a sample.

An alternative method uses the antibodies bound to, for example, fluorescently labeled beads such as commercially available Luminex™ beads. Different beads are used with different antibodies. Different beads are labeled with different fluorophore mixtures, thus allowing different analytes to be determined by the fluorescent wavelength. Luminex beads are available from Luminex Corporation, Austin, Texas, United States of America.

Preferably the assay used is a nephelometric or turbidimetric method. Nephelometric and turbidimetric assays for the detection of λ - or κ - FLC and total FLC are generally known in the art. They have the best level of sensitivity for the assay. λ and κ FLC concentrations may be separately determined or a single assay for total FLC arrived at. Such an assay contains anti-κ and anti-λ FLC antibodies typically at a 60:40 ratio, but other ratios, such as 50:50 may be used.

Antibodies may also be raised against a mixture of free λ and free κ light chains.

Preferably the assay is capable of determining FLC, for example total FLC, in the sample for example from approximately 1 mg/L to 100 mg/L, or 1 mg/L - 80 mg/L. This detects the serum FLC concentrations in the vast majority of individuals without the requirement for re-assaying samples at a different dilution.

Preferably the method comprises detecting the amount of total free light chain in the sample utilising an immunoassay, for example, by utilising a mixture of anti-free κ light chain and anti-free λ light chain antibodies or fragments thereof. Such antibodies may be in a ratio of 50:50 anti-κ: anti-λ antibodies. Antibodies, or fragments, bound to FLC may be detected directly by using labelled antibodies or fragments, or indirectly using labelled antibodies against the anti-free λ or anti-free κ antibodies.

The antibodies may be polyclonal or monoclonal. Polyclonal may be used because they allow for some variability between light chains of the same type to be detected as they are raised against different parts of the same chain. The production of polyclonal antibodies is described, for example in WO97/17372.

Commercial assay kits for measuring FLC are available (Freelite™ and Combylite™, The Binding Site Group Limited, Birmingham, United Kingdom).

Assay kits are provided for use in a method according to the invention as defined in claim 10, comprising one or more anti-FLC antibodies (or fragments) and one or more anti-EBV antibodies or fragments specific for EBV. Antigens which are markers are generally known for EBV. Antibodies may be provided for such markers to further characterise the disorder.

The assay kits may be adapted to detect an amount of total free light chain (FLC) in a sample below 25 mg/L, most preferably, below 20 mg/L or about, 10 mg/L, below 5 mg/L or 4 mg/L. The calibrator material typically measures the range 1-100mg/L. The assay kit may be, for example, a nephelometric assay kit. Preferably the kit is an immunoassay kit comprising one or more antibodies against FLC. Typically the kit comprises a mixture of anti-κ and anti-λ FLC antibodies. Typically a mixture of 50:50 anti-free κ and anti-free λ antibodies are used. The kit may be adapted to detect an amount of 1 - 100 mg/L, or preferably 1 - 80 mg/L total free light chain in a sample.

Fragment of antibodies, such as (Fab)₂ or Fab antibodies, which are capable of binding FLC or EBV as appropriate may also be used.

The antibodies or fragments may be labelled, for example with a label as described above. Labelled anti-immunoglobulin binding antibodies or fragments thereof may be provided to detect anti-free λ or anti-free κ bound to FLC.

The kit may comprise calibrator fluids to allow the assay to be calibrated at the ranges indicated. The calibrator fluids preferably contain predetermined concentrations of FLC, for example 100mg/L to 1mg/L, below 25 mg/L, below 20 mg/L, below 10 mg/L, below 5 mg/L or to 1 mg/L. The kit may also be adapted by optimising the amount of antibody and "blocking" protein coated onto the latex particles and, for example, by optimising concentrations of supplementary reagents such as polyethylene glycol (PEG) concentrations. The kit may comprise, for example, a plurality of standard controls for the FLC. The standard controls may be used to validate a standard curve for the concentrations of the FLC or other components to be produced. Such standard controls confirm that the previously calibrated standard curves are valid for the reagents and conditions being used. They are typically used at substantially the same time as the assays of samples from subjects. The standards may comprise one or more standards below 20 mg/L for FLC, more preferably below 15 mg/L, below approximately 10 mg/L or below 5 mg/L, in order to allow the assay to calibrate the lower concentrations of free light chain.

The assay kit may be a nephelometric or turbidimetric kit. It may be an ELISA, flow cytometry, fluorescent, chemiluminescent or bead-type assay or dipstick. Such assays are generally known in the art.

The assay kit may also comprise instructions to be used in the method according to the invention. The instructions may comprise an indication of the concentration of free light chain considered to be a normal value, below which, or indeed above which, shows an indication of either increased or decreased probability of PTLD of the individual or an increased likelihood of PTLD being present, for example, or having a poor prognosis. Such concentrations may be as defined above.

The invention will now be described by way of example only, with reference to the following figures:

The data below is based on a cohort of kidney transplant patients, the Applicant has specifically examined two groups within the cohort. The first is a set of kidney transplant patients (n=351), who after varying although significant follow up had been shown to neither have developed cancer nor specifically PTLD; free light chain levels were determined on a single serum sample taken at different times post transplant, the median follow up time after sample collection was around 1700 days. The second set of kidney transplant patients (n=5), had developed PTLD at some time point during follow up; free light chain levels were determined on a single serum sample taken after transplant, PTLD developed in these patients between 314 and 1594 days after sample collection. The median level for combined free light chains in the non-PTLD group was less than 50mg/L whereas in the group of patients that subsequently developed PTLD the median level of combined free light chains was greater than 60 mg/L. The number of PTLD patients was low, however, the difference in the median levels of combined free light chains between the two groups was substantial (>20%).

| **PTLD Patient** | **Days: Transplant to sample** | **Kappa (mg/L)** | **Lambda (mg/L)** | **Total (mg/L)** | **Kappa/Lambda Ratio** | **Days: Sample to PTLD** |
|---|---|---|---|---|---|---|
| 1 | 3121 | 33.30 | 41.80 | 75.10 | 0.80 | 1136 |
| 2 | 2124 | 28.10 | 31.00 | 59.10 | 0.91 | 314 |
| 3 | 662 | 16.50 | 14.40 | 30.90 | 1.15 | 1101 |
| 4 | 589 | 25.80 | 36.50 | 62.30 | 0.71 | 1594 |
| 5 | 742 | 19.10 | 56.50 | 75.60 | 0.34 | 964 |
| | | | | | | |
| Median | | 25.8 | 36.5 | 62.3 | | 1101 |

Analysis of the total FLC levels in the two populations (351 Tx patients and 5 Tx with PTLD) using distribution analysis (Independent samples Wald-Wolfowitz Runs test) and comparison of Medians (Independent samples median test) showed that there was a significantly different distribution between the two groups (p<0.001) and therefore it would be unlikely that an observation seen in the control arm may be present in the test arm. The median values analysis showed a tendency towards significance (p=0.175), which reflects the low number of PTLD patients samples tested.

## Claims

1. A method of identifying the risk of an organ transplant patient developing post transplant lymphoproliferative disorder (PTLD), identifying the likelihood of the patient having PTLD, or identifying a prognosis of a patient with PTLD, comprising detecting an amount of free light chains (FLC) in a sample from the patient, wherein a higher than normal amount of FLC or abnormal FLC ratio in the sample, indicates an increased risk of developing PTLD, having PTLD or having a poor prognosis for the PTLD.

2. A method of monitoring the progress of PTLD in a patient, treatment of patients with increased risk of developing PTLD, or monitoring the treatment of PTLD in a patient comprising detecting an amount of FLC in a sample from the patient and comparing it to an amount of FLC from a previously taken sample from the patient.

3. A method according to any preceding claim wherein the amount of FLC detected is selected from the amount of λ FLC, the amount of κ FLC, the total amount of FLC and the κ:λ FLC ratio in the sample.

4. A method according to any preceding claim wherein the patient has received a solid organ from a donor.

5. A method according to claims 1 to 4, wherein the FLC is determined in a sample of urine, serum, blood or plasma from the subject.

6. A method according to claims 1 to 5, wherein the total FLC is determined by immunoassay using anti-free light chain antibodies.

7. A method according to claim 6, wherein the antibodies are a mixture of anti-free κ light chain and anti-free λ light chain antibodies.

8. A method according to claims 1 to 7, wherein the method comprises detecting the amount of FLC by nephelometry or turbidimetry.

9. A method according to claims 1 to 8, wherein the subject did not have symptoms of a B-cell associated disease, prior to transplant.

10. An assay kit for use in the method according to claim 1 to 8 additionally comprising instructions to be used in the method and one or more anti-FLC antibodies or fragments thereof capable of binding FLC and one or more anti-Epstein Barr Virus (EBV) antibodies or fragments thereof, capable of binding EBV.

11. An assay kit according to claim 10 additionally comprising a normal value against which a concentration of FLC obtained using the assay kit, indicates an increased risk of developing PTLD or having PTLD.

## Patentansprüche

1. Verfahren zum Feststellen des Risikos, dass ein Organtransplantationspatient eine lymphoproliferative Erkrankung nach Transplantation (PTLD) entwickelt, Feststellen der Wahrscheinlichkeit, dass der Patient PTLD hat, oder Feststellen einer Prognose eines Patienten mit PTLD, umfassend ein Erfassen einer Menge freier Leichtketten (FLC) in einer Probe des Patienten, wobei eine höhere als die normale Menge an FLC oder ein anormales FLC-Verhältnis in der Probe ein erhöhtes Risiko anzeigt, PTLD zu entwickeln, PTLD zu haben oder eine schlechte Prognose für die PTLD zu haben.

2. Verfahren zur Überwachung des Fortschreitens von PTLD bei einem Patienten, Behandlung von Patienten mit erhöhtem Risiko, PTLD zu entwickeln, oder Überwachung der Behandlung von PTLD bei einem Patienten, umfassend ein Erfassen einer Menge an FLC in einer Probe des Patienten und Vergleichen derselben mit einer Menge an FLC aus einer früher genommenen Probe des Patienten.

3. Verfahren nach einem der vorstehenden Ansprüche, wobei die erfasste Menge an FLC ausgewählt ist aus der Menge an λ-FLC, der Menge an κ-FLC, der Gesamtmenge an FLC und dem Verhältnis von κ-FLC : λ-FLC in der Probe.

4. Verfahren nach einem der vorstehenden Ansprüche, wobei der Patient ein Festorgan von einem Spender erhalten hat.

5. Verfahren nach Anspruch 1 bis 4, wobei die FLC in einer Urin-, Serum-, Blut- oder Plasmaprobe des Subjektes bestimmt werden.

6. Verfahren nach Anspruch 1 bis 5, wobei die Gesamt-FLC durch Immunassay mit Antikörpern gegen freie Leichtketten bestimmt werden.

7. Verfahren nach Anspruch 6, wobei die Antikörper eine Mischung von Antikörpern gegen freie κ-Leichtketten und freie λ-Leichtketten ist.

8. Verfahren nach Anspruch 1 bis 7, wobei das Verfahren ein Erfassen der Menge an FLC durch Nephelometrie oderTurbidimetrie umfasst.

9. Verfahren nach Anspruch 1 bis 8, wobei das Subjekt vor der Transplantation keine Symptome einer B-Zellen-assoziierten Erkrankung aufwies.

10. Assay-Kit zur Verwendung in dem Verfahren nach Anspruch 1 bis 8, zusätzlich umfassend:
Anweisungen zur Verwendung in dem Verfahren und einen oder mehr Antikörper gegen FLC oder Fragmente davon mit der Fähigkeit zum Binden von FLC und einen oder mehr Antikörper gegen das Epstein-Barr-Virus (EBV) oder Fragmente davon mit der Fähigkeit zum Binden von EBV.

11. Assay-Kit nach Anspruch 10, das zusätzlich einen Normalwert umfasst, gegenüber dem eine mit dem Assay-Kit erhaltene FLC-Konzentration ein erhöhtes Risiko anzeigt, PTLD zu entwickeln oder PTLD zu haben.

## Revendications

1. Procédé permettant d'identifier le risque qu'un patient ayant subi une transplantation d'organe développe une affection lymphoproliférative post transplantation (PTLD), d'identifier la probabilité que le patient développe une PTLD, ou d'identifier la prévision qu'un patient soit atteint d'une PTLD, comprenant une étape consistant à détecter la quantité de chaînes légères libres (FLC) dans un échantillon du patient, une quantité de FLC supérieure à la normale, ou un rapport de FLC anormal dans l'échantillon indiquant un risque augmenté de développer une PTLD, d'avoir une PTLD ou d'avoir une mauvaise prévision de développement d'une PTLD.

2. Procédé permettant de surveiller la progression d'une PTLD chez un patient, de traiter des patients ayant un risque augmenté de développer une PTLD ou de surveiller le traitement d'une PTLD chez un patient comprenant une étape consistant à détecter la quantité de FLC dans un échantillon du patient et à la comparer à la quantité de FLC dans un échantillon du patient prélevé antérieurement.

3. Procédé conforme à l'une quelconque des revendications précédentes selon lequel la quantité de FLC détectée est choisie parmi la quantité de λ, FLC, la quantité de κ FLC, la quantité de FLC totale et le rapport FLC κ : FLC λ dans l'échantillon.

4. Procédé conforme à l'une quelconque des revendications précédentes selon lequel le patient a reçu un organe solide d'un donneur.

5. Procédé conforme aux revendications 1 à 4, selon lequel la FLC est déterminée dans un échantillon d'urine, de sérum, de sang ou de plasma du sujet.

6. Procédé conforme aux revendications 1 à 5, selon lequel la FLC totale est déterminée par immuno test en utilisant des anticorps anti chaînes légères libres.

7. Procédé conforme à la revendication 6, selon lequel les anticorps sont un mélange d'anticorps anti chaînes légères κ et d'anticorps anti chaînes légères λ.

8. Procédé conforme aux revendications 1 à 7 comprenant une étape consistant à détecter la quantité de FLC par néphélométrie ou turbidimétrie.

9. Procédé conforme aux revendications 1 à 8, selon lequel le sujet ne présentait pas de symptôme d'une affection associée aux cellules B avant la transplantation.

10. Kit de test destiné à être utilisé pour la mise en oeuvre du procédé conforme à l'une des revendications 1 à 8 comprenant en outre des instructions devant être utilisées lors de la mise en oeuvre du procédé et un ou plusieurs anticorps anti FLC ou fragments de tels anticorps susceptibles de lier les FLC et un ou plusieurs anticorps anti Epstein Barr Virus (EBV) ou fragments de tels anticorps susceptibles de se lier à EBV.

11. Kit de test conforme à la revendication 10 comprenant en outre une valeur normale par rapport à laquelle une concentration de FLC obtenue en utilisant le kit de test indique un risque augmenté de développer une PTLD ou d'avoir une PTLD.
